# EUROPEAN PATENT SPECIFICATION

(11) **EP 3 191 456 B1**
(45) Date of publication and mention of the grant of the patent: **17.04.2019**
(21) Application number: 15787030.4
(22) Date of filing: 11.09.2015
(51) Int. Cl.: C07D 263/14

(54) **A ONE POT PROCESS FOR SYNTHESIS OF OXAZOLINE COMPOUNDS FROM GLYCEROL**
EINTOPFVERFAHREN ZUR SYNTHESE VON OXAZOLINVERBINDUNGEN AUS GLYCERIN
PROCÉDÉ MONOTOPE DE SYNTHÈSE DE COMPOSÉS D'OXAZOLINE À PARTIR DE GLYCÉROL

(30) Priority: 12.09.2014 IN 2621DE2014
(43) Date of publication of application: 19.07.2017
(73) Proprietor: Council of Scientific and Industrial Research, New Delhi, Maharashtra 110001 (IN)
(72) Inventor: RODE, Chandrashekhar Vasant, Pune Maharashtra 411 008 (IN); PANDYA, Rajan, Pune Maharashtra 411 008 (IN); MANE, Rasika Bharat, Pune Maharashtra 411 008 (IN)
(74) Representative: HGF Limited
(86) International application number: PCT/IN2015/050108
(87) International publication number: WO 2016/038632

(56) References cited:
- EP-A1- 0 252 162
- WO-A1-2007/146144
- WO-A2-2011/138643
- FR-A1- 2 328 701
- YANG CHUNHUA ET AL: "Study of acetol conversion to 4-methylimidazole", YINGYONG-HUAGONG = APPLIED CHEMICAL INDUSTRY, YINGYONG HUAGONG ZAZHISHE, CN, vol. 39, no. 5, 1 January 2010 (2010-01-01), pages 783-786, XP009187389, ISSN: 1671-3206

## Description

### FIELD OF THE INVENTION:

The present invention relates to a single step one pot process for synthesis of nitrogen containing heterocyclic compounds from glycerol. More particularly, the present invention relates to a single step one pot process for synthesis of oxazoline derivatives from glycerol using solid acid metal catalyst with improved conversion and selectivity.

### BACKGROUND AND PRIOR ART OF THE INVENTION:

Oxazolines are five-membered cyclic compounds having an endo-imino ether (AN¼¼CAOA) group. They belong to one of the classes of cyclic imino ethers, which contain cyclic endo- and exo-imino ethers of 5-, 6-, and 7-membered rings. An early stage of chemistry of oxazolines including their preparation, reactions, and applications was extensively reviewed previously. Historically, oxazolines have been known since the end of 19th century. The first attempt of the preparation was made in 1884 via dehydrohalogenation of allyl urea, resulting in detection of a new cyclic compound but failing to deduce its correct chemical structure. Five years later, the first successful synthesis of oxazolines was reported and then the chemistry of oxazolines started to be increasingly investigated.

Oxazolines have three structural isomers; 2-oxazoline, 3-oxazoline, and 4-oxazolines. Among them, 2-oxazolines are by far the most well-known and extensively studied. The formation of an oxazoline ring was postulated in the cyclodehydration at peptide linkages constituted from the a-amino-b-hydroxy acids, serine, or threonine. 2-Substituted-2-oxazolines were formed via the cyclization of an acyl derivative of a-amino acid with dehydration, the reaction of an amino alcohol with a carboxylic acid, the cyclization of a b-haloalkylamide via dehydrohalogenation, and so forth.Chemistry of these hetero-cyclic compounds was widely studied by reactions with a variety of compounds. Oxazoline is used in polymer chemistry, to make amino alcohols, is stable, starting compound for making ionic liquids, pharma-compound that reduces appetite and facilitates weight loss.

U.S. patent no. 4105669 discloses manufacture of 2-amino-1-alcohols carried out by reacting the hydroxyketone with ammonia at from about 0° to 120° C, preferably at from room temperature 20° C to 80° C, at a pressure of from atmospheric pressure to about 300 bars, preferably up to about 100 bars. The reaction may be carried out without using extraneous solvents. But reaction takes place in two steps with less conversion and selectivity about less than 85 percent and also reaction took place in harsh conditions.

U.S. patent no. 7619119 discloses two steps process for converting glycerol to an amino alcohol product involving reacting glycerol with a metal catalyst to obtain hydroxyacetone and reacting the hydroxyacetone with an amine compound to obtain an adduct that is then reduced using a reducing agent to obtain an amino alcohol product. The metal catalyst is a catalyst selected from the group consisting of copper, chromium, nickel, zinc, cobalt, manganese, silicon, aluminum, copper chromite, copper zinc, oxides thereof, and combinations of any thereof.

U.S. patent application no. 20080045749 discloses two steps industrial process for the alternating production of propylene glycol or an amino alcohol product from glycerol comprising: 1) reacting glycerol with a metal catalyst to obtain hydroxyacetone;2)optionally reacting the hydroxyacetone with an amine compound to obtain an adduct; and 3) reducing the hydroxyacetone or the adduct using a reducing agent to obtain a product, wherein the product is propylene glycol when the hydroxyacetone is reduced with the reducing agent and the product is an amino alcohol product when the adduct is reduced with the reducing agent. The metal catalyst is a catalyst selected from the group consisting of copper, chromium, nickel, zinc, cobalt, manganese, silicon, aluminum, copper chromite, copper zinc, oxides thereof, and combinations of any thereof.

U.S. patent no. 8809593 discloses a process for the preparation of the hydroxyacetone or 1,2 propylene glycol. This process is catalyzed by metal catalysts that results in 80 to 100% selectivity towards conversion of glycerol to hydroxyacetone (acetol) or 1,2 propylene glycol. The metal catalysts are selected from the group Cu, Cr, Al, Ba, Zn, Si, Zr, Mg or in combinations thereof.

Article titled "Study on the reactions of ammonia precursors and alpha-hydroxy carbonyl compounds (iii), identification of novel 3-oxazolines" by Ken Shu et al. product formulation. PD93 188, 7th July 1993. The major compounds formed from the reactions between alpha- hydroxy carbonyls and ammonium hydroxide has been identified by MS, IR, NMR and synthesis as 3-oxazolines.

Article titled "Simultaneous glycerol dehydration and in situ hydrogenolysis over Cu-Al oxide under an inert atmosphere" by Rasika Mane et al. published in Green Chemistry, 2012,14, 2780-2789 reports dehydration of glycerol and in situ hydrogenolysis in presence of Cu-Al oxide catalyst. Substantial enhancement in 1,2-Propane diol selectivity (75%) was achieved for an aqueous bio-glycerol feed over the same catalyst for 50 h of testing.

Article titled "Continuous dehydration and hydrogenolysis of glycerol over non-chromium copper catalyst: laboratory-scale process studies" by Rasika Mane et al. published in Organic Process Research & Development, 2012, 16(5):1043-1052 reports a non-chromium Cu:Al catalyst was developed for glycerol dehydration under N2 atmosphere to acetol and hydrogenolysis to 1,2-propanediol (1,2-PDO). Among the various copper-based catalysts screened in this work, Cu:Al-1 catalyst showed the highest activity and acetol selectivity in water medium.

Article titled "Dehydration of glycerol to acetol via catalytic reactive distillation" by Chuang-Wei Chiu et al. published in AIChE Journal, 2006, 52 (10), 3543-3548 reports dehydration of glycerol in the presence of various metallic catalysts including alumina, magnesium, ruthenium, nickel, platinum, palladium, copper, raney nickel, and copper-chromite catalysts to obtain acetol in a single-stage reactive distillation unit under mild conditions. High-acetol selectivity levels (> 90%) were achieved using copper-chromite catalyst, and operating in semi-batch reactive distillation mode.

Article titled "Thermal conversion of glycerol to value-added chemicals: pyridine derivatives by one-pot microwave-assisted synthesis" by DuyguBayramoglu et al. published in Turkish Journal of Chemistry, 2014, 38: 661 - 670 reports one-pot syntheses of the value-added heterocyclic compounds 3-methylpyridine and pyridine using a renewable chemical, glycerol, were achieved in acidic medium by thermal conversion reactions. Condensation/cyclization reactions of the thermal degradation products of glycerol were investigated in situ using different ammonia and acidic moiety producing inorganic ammonium salts under pyrolysis or microwave heating conditions. But reaction takes place in harsh conditions.

Article titled "A review of acetol: application and production" by MohdHanif Mohamad et al. published in American Journal of Applied Sciences, 2011, 8 (11), pp 1135-1139 reports the production of acetol from glycerol is greatly influenced by the presence of catalyst. The most famous catalyst used in the production of acetol from glycerol is the metal supported acidic catalyst. In nitrogen flow, unreduced Cu-Al2O3 decreases the conversion and increases acetol selectivity with time on stream. Meanwhile, when CuAl2O3 was subjected to hydrogen reduction, it is fully converted to glycerol and acetol selectivity increases with time on stream for the first 5 hours.

Article titled "Study of acetol conversion to 4-methylimidazole" by Yang Chunhua et al published in Applied Chemical Industry, vol 39, no.5, 1 January 2010, pages 783-786 relates to the production of 4-methlimidazole from acetol.

The above reported processes in prior arts involve multi steps with low yields. Therefore there is a need to develop a single step one pot process with high yields.

### OBJECTIVE OF THE INVENTION:

The main objective of the present invention is to provide a single step one pot process for synthesis of nitrogen containing heterocyclic compounds from glycerol using solid acid metal catalyst with high yields.

### SUMMARY OF THE INVENTION:

In a first aspect of the present invention, there is provided a one pot process for the synthesis of oxazoline compounds as defined in claim 1. Herein is disclosed a single step one pot process for synthesis of nitrogen containing heterocyclic compounds from glycerol using solid acid metal catalyst with high yields.

Herein is disclosed a single step, one pot process for synthesis of nitrogen containing heterocyclic compounds from glycerol or bio- glycerol using solid acid metal catalysts comprising the steps of:
a. stirring the mixture of glycerol and solid acid metal catalyst at a temperature in the range of 180 to 240°C for a period in the range of 4-6 hrs to obtain acetol;
b. cooling the reaction mixture of step (a) to a temperature in the range of 0°C to 50°C and adding ammonia solution followed by stirring the mixture for the period in the range of 1-10 hrs to obtain nitrogen containing heterocyclic compound; wherein, the selectivity of said heterocyclic compound is greater than 95%.

In an embodiment, the present invention provides a single step, one pot process for synthesis of oxazoline from glycerol or bio-glycerol using solid acid metal catalyst with high yields comprising the steps of:
(i) stirring the mixture of glycerol and solid acid metal catalyst at a temperature in the range of 180 to 240°C for a period in the range of 4-6 hrs to obtain acetol;
(ii) cooling the reaction mixture of step (a) to a temperature in the range of 0°C to 50°C and adding ammonia solution followed by stirring the mixture for a period in the range of 2-10 hrs to obtain desired oxazoline.

In yet another embodiment, the present invention provides a metal catalyst selected from Cu, Al, Zr, Zn, Mg, Ba, Ru, Pd, Pt or combinations thereof.

In preferred embodiment, the present invention provides nitrogen containing heterocyclic compounds is oxazoline.

In another embodiment, the present invention provides a process which optionally comprises addition of water to a mixture of glycerol and solid acid metal catalyst.

In still another embodiment, the present invention provides stirring in step (b), which is carried out at temperature in the range of 10°C to 20°C.

In an embodiment, the metal catalyst is selected from combinations of Cu- Al, Cu- Zr or Cu- Mg, Ru/C, Ru/s-ZrO₂,Ru/γ-Al₂O₃.

In another embodiment, the ratio of glycerol: ammonia is in the range of 1:1 to 1:3.

The selectivity of oxazoline prepared by said process is >95%. More preferably the selectivity is >97%.

In yet another embodiment, said process is carried via reactive distillation or batch mode.

### BRIEF DESCRIPTION OF THE DRAWINGS:

**Figure 1****:** Reactive distillation set-up

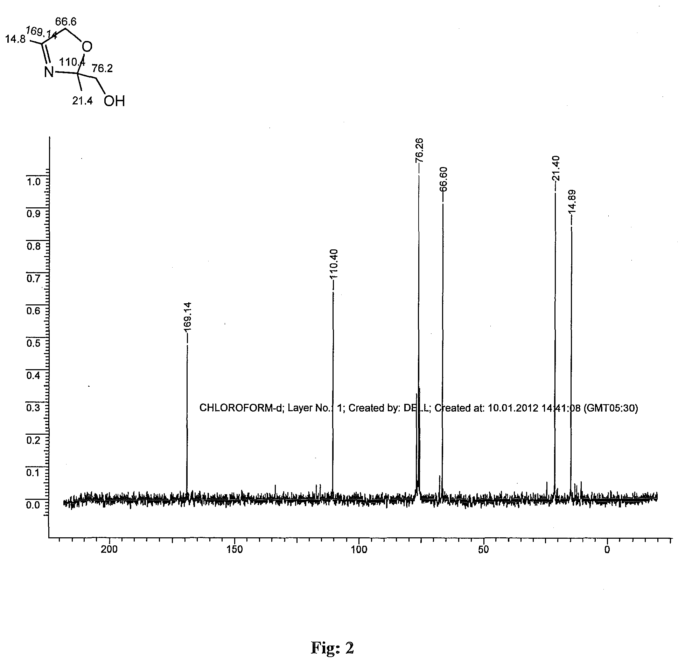

**Figure 2****:**¹³C NMR of oxazoline

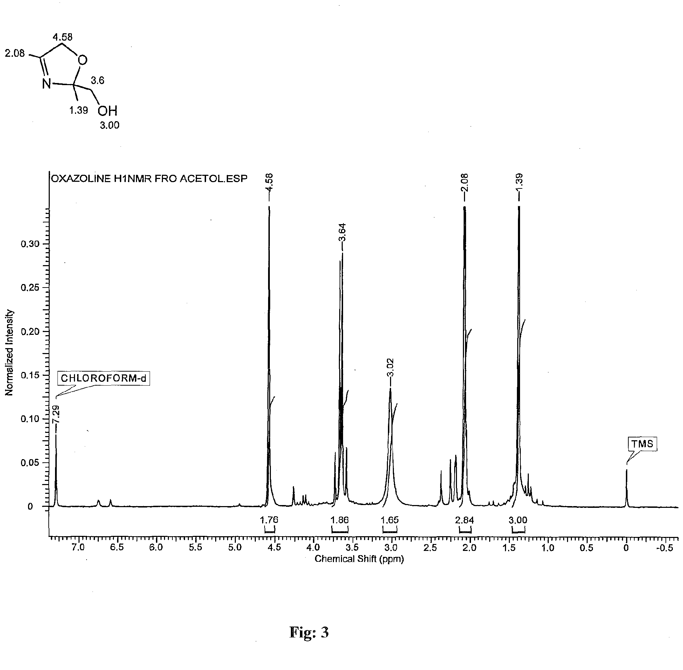

**Figure 3****:** ¹H NMR of oxazoline

### DETAILED DESCRIPTION OF THE INVENTION:

The invention will now be described in detail in connection with certain preferred and optional embodiments, so that various aspects thereof may be more fully understood and appreciated.

In the view of above, the present invention provides a single step one pot process for synthesis of oxazoline from glycerol using solid acid metal catalyst with high yield as defined in claim 1.

In an embodiment, the present invention provides a single step, one pot process for the synthesis of oxazoline from glycerol using metal catalysts with selectivity greater than 95%, comprising reacting glycerol in the presence of metal catalyst, cooling, adding ammonia and stirring the reaction mixture to obtain oxazoline as defined in claim 1.

The present invention provides Scheme 1:

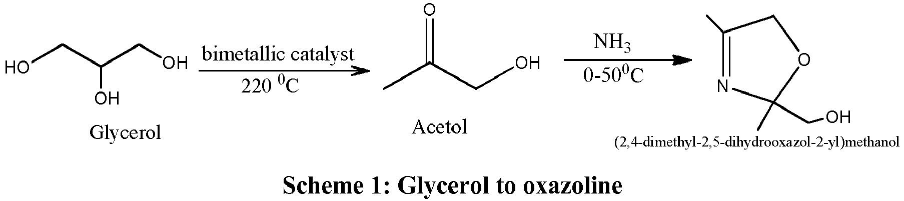

In preferred embodiment, the present invention provides a process wherein the metal catalyst is selected from Cu, Al, Zr, Zn, Mg, Ba, Ru, Pd, Pt or combinations thereof.

In more preferred embodiment, the present invention provides a process wherein the metal catalyst is a bimetallic catalyst preferably selected from combinations of Cu- Al, Cu-Zr or Cu- Mg.

In another preferred embodiment, said metal catalyst is selected from Ru/C, Ru/s-ZrO₂,Ru/γ-Al₂O₃.

In another preferred embodiment, the present invention provides a process wherein the ratio of glycerol: ammonia is in the range of 1:1 to 1:3; reaction temperature is in the range of 180-220°C; cooling temperature is in the range of 0-50°C.

The present invention provides a process wherein the selectivity is >95% and preferably >97%.

In yet another preferred embodiment, the present invention provides a process wherein the reaction can be carried via reactive distillation or batch mode. More preferably, the reaction is carried out via reactive distillation.

Herein is described a process for the preparation of imidazole derivatives from glycerol comprising:
a. carrying the dehydration reactions in a stirred autoclave of bio-glycerol and water in presence of metal catalyst at ambient pressure of nitrogen to obtain the reaction mixture;
b. filtering and cooling the reaction mixture of step (a) followed by addition of ammonia, metal catalyst and stirring to obtain imidazole derivatives.

The above process is shown below in Scheme 2

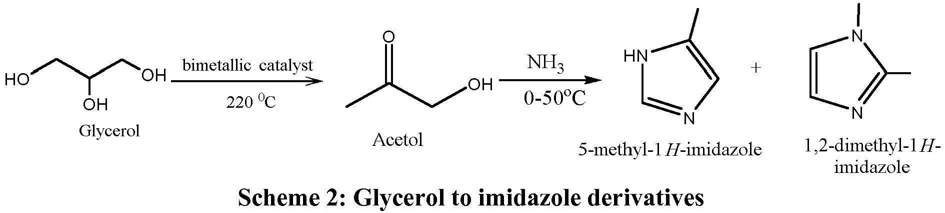

In preferred embodiment, the present invention provides a process wherein the metal catalyst is selected from Cu, Al, Zr, Zn, Mg, Ba, Ru, Pd, Pt or combinations thereof

In more preferred embodiment, the present invention provides a process wherein the metal catalyst is a bimetallic catalyst preferably selected from combinations of Cu- Al, Cu-Zr or Cu- Mg.

In another preferred embodiment, the present invention provides a process wherein the reaction is carried via batch mode.

In yet another preferred embodiment, the present invention provides a process wherein the ratio of glycerol: ammonia is in the range of 1:1 to 1:3; reaction temperature is in the range of 180-220°C; cooling temperature is in the range of 0-50°C.

The present invention provides a process wherein the selectivity is >95% and preferably >97%.

A process for the preparation of the metal catalyst forms part of the disclosure.

In a preferred aspect, the invention provides copper catalysts with various metals such as Zr and Al which are prepared by co-precipitation method with simultaneous addition of equimolar (0.05 M) mixture of aqueous solution of Cu (NO₃)₂.3H₂O, nitrate precursor of a respective metal and 0.2 M aqueous K₂CO₃ in a round bottom flask having 5-10 mL of water at room temperature. The obtained precipitate is digested for 4-5 h and then filtered and washed with deionized water to remove the traces of potassium. The precipitate is dried in an oven at 100 °C for 5-8 h and calcined at 400 °C for 3 h followed by its activation at 200 °C under H₂ flow for 12 h.

The following examples, which include preferred embodiments, will serve to illustrate the practice of this invention, it being understood that the particulars shown are by way of example and for purpose of illustrative discussion of preferred embodiments of the invention.

### EXAMPLES:

The experiments with reactive distillation were carried out in a glass reactor capacity of 100 mL. A magnetic stirrer with an agitation speed of 600 rpm was used to create a slurry reaction mixture. A condenser was attached to the top of a glass reactor, through which chilled water was circulated. In a typical procedure, the glass reactor was immersed in a constant temperature silicon oil batch, the temperature of which was maintained at 220 °C. In the glass reactor, the catalyst and reaction mixture were first heated to the reaction temperature of 220 °C. The distillate was continuously collected in a glass receiver connected to the condenser which was maintained at 0 °C using ice bath. All the experiments were conducted under vacuum by using a vacuum pump.

### Example 1: Preparation of catalyst: (catalyst preparation repeated twice):

a) Copper catalysts with various metals such as Zr and Al were prepared by co-precipitation method with simultaneous addition of equimolar (0.05 M) mixture of aqueous solution of Cu (NO₃)₂.3H₂O, nitrate precursor of a respective metal and 0.2 M aqueous K₂CO₃ in a round bottom flask having 5-10 mL of water at room temperature. The obtained precipitate was digested for 4-5 h and then filtered and washed with deionized water to remove the traces of potassium. The precipitate was dried in an oven at 100 °C for 5-8 h and calcined at 400°C for 3 h followed by its activation at 200 °C under H₂ flow for 12 h.
b) Supported Ru, Pd, Pt catalysts were prepared by impregnation method. The synthesis was performed by suspending 2 g of activated carbon in aqueous medium using calculated amount of the respective metal precursors and then suspension was stirred for 2 h. It was then subsequently reduced using 5 mL of NaBH₄ (1 mol) as a reducing agent. The catalyst was filtered and dried at 110 °C for 12 h.

### Example 2: Preparation of oxazoline [2,4-dimethyl-2,5-dihydrooxazol-2-yl)methanol] from Glycerol:

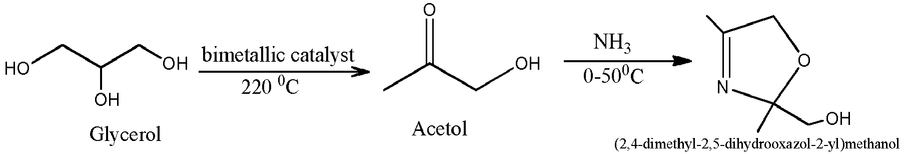

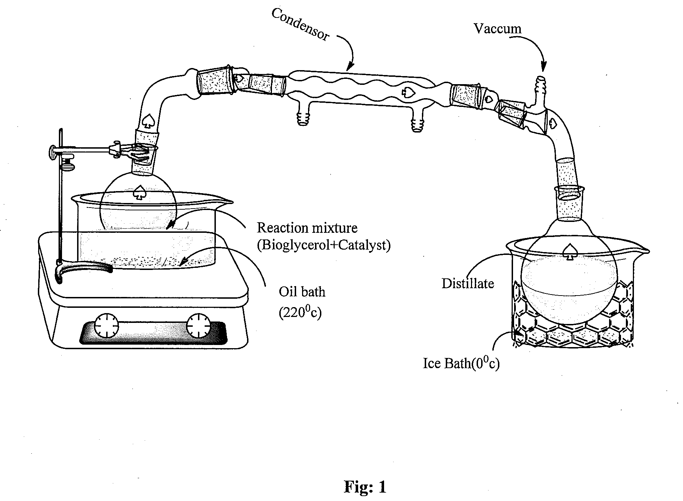

The reaction was carried out in reactive distillation set up (Figure 1) in which round bottom flask having 100 mL capacity was taken to which 50 g of pure bio-glycerol and 0.8 g of Cu-Al catalyst were added (Figure 1). This reaction mixture was stirred by a magnetic stirrer at an agitation speed of 600 rpm and temperature was maintained at 220 °C by using silicon oil bath. The reaction was continued for about 6 h and acetol formed was continuously distilled out during the reaction. The obtained distillate mainly containing acetol and 1, 2-propanediol, was analyzed by GC (Model Shimadzu GC-2025) equipped with an auto sampler (Model AOC-20i) and a capillary FFAP (Free Fatty Acid Phase) (30 m length×0.53mm i.d. × 1µm film thickness) column connected to the flame ionization (FID) detector. The distillate was cooled to 0-10°C and then 30% aqueous ammonia solution (1:3 ratio) was added drop wise with stirring in the presence of 0.05g Cu-Al catalyst,

The reaction mixture was stirred for 1 h at 50 °C and reaction progress was monitored by GC. (Fig: 2 and 3)

13C NMR (200MHz, CHLOROFORM-d) δ ppm: 14.89, 21. 40, 66.6, 76.26, 110.40, 169.14. 1HMR (200MHz, CHOLOROFORM-d) δ ppm: 1.39(CH₃,s,3H), 2.08 (CH₃,s,3H), 3.02(OH,s,1H), 3.64(CH₂,d,2H), 4.58(CH₂,s,2H).

**Table 1** depicts glycerol dehydration to acetol by reactive distillation and its amination to oxazoline.

**Table 1:**

| Sr.No. | Cata. | **Glycerol to Acetol** | | | | **Acetol to Oxazoline** | | | |
|---|---|---|---|---|---|---|---|---|---|
| | | Conv. | Selectivity | | | Conv. | Selectivity | | |
| | | | Acetol | 1-2,PDO | Solketal | | Oxazoline | 5-methyl imidazole | 1,2-dimethyl imidazole |
| 1 | Cu-Al | 20 | 80 | 7 | 13 | 99 | 95 | 3 | 2 |
| 2 | Cu-Zr | 22 | 82 | 8 | 10 | 99 | 95 | 3 | 2 |
| 3 | Cu-Mg | 25 | 75 | 10 | 15 | 99 | 95 | 3 | 2 |

**Reaction conditions: -Glycerol to Acetol-** 50 g bio glycerol, T= 220°C, catalyst = 0.8 g, reaction time = 6 h; **Acetol to oxazoline-** 10 g distillate, T= 50, catalyst = 0.01 g, reaction time = 2 h.

### Reference Example 3 not forming part of the invention: Preparation of imidazole derivatives from Glycerol:

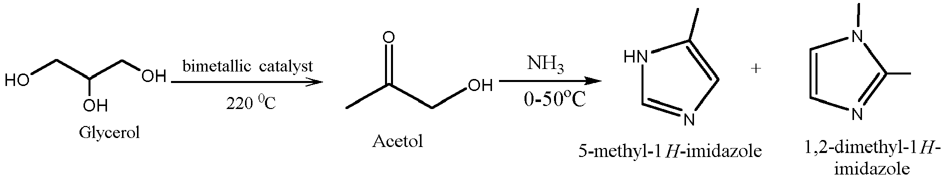

The dehydration reactions were carried out in a 300 mL capacity stirred autoclave supplied by Parr Instruments Co.USA., in which 5% W/w of bio-glycerol and 95 ml of water is added along with 0.8 gm of catalyst at ambient pressure of nitrogen. The reactions were carried out for 3 h and then catalyst is filtered out by using filter paper. The filtrate was cooled to 0-10 °C and then 30% aqueous ammonia solution (15 mL) was added drop wise with stirring,
and then the reaction mixture was stirred for 2 h at 50°C and reaction progress was monitored by GC.

**Table 2** depicts glycerol dehydration to acetol in batch reactor followed by its amination to imidazole.

**Table 2:**

| Sr. No. | Cata. | **Glycerol to acetol** | | | | **Acetol to imidazole** | | | |
|---|---|---|---|---|---|---|---|---|---|
| | | Conv. (%) | Selectivity (%) | | | Conv. (%) | Selectivity (%) | | |
| | | | Acetol | 1-2,PDO | other | | 5-methyl imidazole | 1,2-dimethyl imidazole | oxzoline |
| 1 | Cu-Al | 26 | 91 | 8 | 1 | 100 | 50 | 47 | 3 |
| 2 | Cu-Zr | 22 | 89 | 10 | 1 | 100 | 49 | 45 | 6 |
| 3 | Cu- Mg | 24 | 75 | 20 | 5 | 100 | 49 | 46 | 5 |

**Reaction conditions:Glycerol to Acetol-** 5 wt.% aq. bio glycerol, T= 220°C, catalyst = 0.8 g, reaction time = 3 h ; **Acetol to imidazole** - T= 50 °C, reaction time = 2 h.

### Example 4: Glycerol to oxazoline:

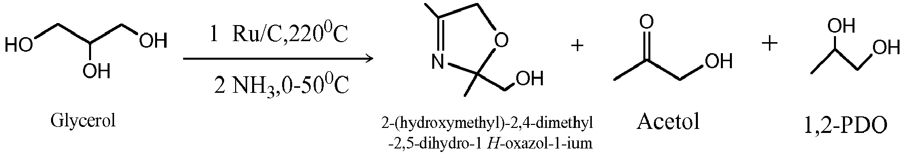

The reactions were carried out in a 300 mL capacity stirred autoclave supplied by Parr Instruments Co.USA., in which 20% W/w of bio-glycerol and 80 ml of water is added along with 0.8 gm of catalyst. The reactions were carried out for 3 h at 220°C. After completion of reaction; reactor is cool down to the 0-10°C by ice chilling water and then 30% Aq. Ammonia solution (10ml) was pumped into the reactor by applying the vacuum. After complete addition of ammonia, the reaction was carried out at 50°C for 1 hr at 1000 rpm. The intermittent liquid samples were analyzed by GC (Model Shimadzu GC-2025) equipped with an auto sampler (Model AOC-20i) and FID detector on a capillary FFAP (Free Fatty Acid Phase) (30m length×0.53mm i.d. × 1µm film thickness) column. The result of one pot synthesis of oxazoline from glycerol with different catalyst is given in table 3.

**Table 3** depicts one pot synthesis of oxazoline from glycerol.

**Table 3:**

| **Sr. No** | **Catalyst** | **Con. (%)** | **Selectivity (%)** | | | | | | |
|---|---|---|---|---|---|---|---|---|---|
| | | | **Oxazoline** | **1,2 PDO** | **Acetol** | **Ethanol** | **EG** | **Imidazole** | **Oth** |
| **1** | 1%Ru/C | 18 | 85 | 3 | 2 | 2 | 1 | 2 | 5 |
| **2** | 3%Ru/C | 26 | 91 | 2 | 1 | 2 | 1 | 2 | 1 |
| **3** | 5%Ru/C | 29 | 78 | 7 | 2 | 4 | 4 | 3 | 2 |
| **4** | 10%Ru/C | 33 | 73 | 10 | 2 | 5 | 5 | 3 | 2 |
| **5** | 3%Pd/C | 14 | 5 | 3 | 3 | 40 | 5 | 40 | 4 |
| **6** | 3%Pt/C | 15 | 5 | 40 | 3 | 20 | 4 | 20 | 4 |
| **7** | 3%Ru/s-ZrO₂ | 22 | 70 | 10 | 2 | 10 | 0 | 2 | 5 |
| **8** | 3%Ru/γ-Al₂O₃ | 20 | 80 | 5 | 3 | 3 | 0 | 4 | 3 |

**Reaction conditions:Glycerol to Acetol-** 20wt.% aq. bio glycerol, T= 220°C, catalyst = 0.8 g, reaction time = 3 h;**Acetol to Oxazoline** - T= 50 °C, reaction time = 2 h.

### ADVANTAGES OF INVENTION:

1) A catalytic, single step one pot process for oxazoline preparation with high yields.
2) The process uses the renewable, cheapest bio-glycerol feed stock.
3) Oxazoline is used as surface active agents (lubricant, dispersing pigments), gasoline and lube oil additives (antiknock and anti-icing), corrosion inhibitors, antifoam agents, textile chemicals, pharmaceuticals (Central nervous system regulators are the substituted 2- amino-2-oxazolines. Aminooxazolines, such as 2-amino-5-phenyl-2-oxazoline, have been tested and found very potent in suppressing appetite. Raising blood sugar levels, and exhibit local aesthetic, sedative, vasoconstrictor, blood pressure depressant, and gastric fluid secretion), adhesives and binders, stabilizers for chlorinated hydrocarbons, stabilizers for aqueous formaldehyde solutions, protective films in polish formulations, foam stabilizers, photography and polymer.

## Claims

1. A one pot process for synthesis of nitrogen containing heterocyclic compounds from glycerol or bio- glycerol using solid acid metal catalysts comprising the steps of:
a. stirring the mixture of glycerol and solid acid metal catalyst at temperature in the range of 180 to 240°C for a period in the range of 4-6 hrs to obtain acetol;
b. cooling the reaction mixture of step (a) to a temperature in the range of 0°C to 50°C and adding ammonia solution followed by stirring the mixture for the period in the range of 1-10 hrs to obtain nitrogen containing heterocyclic compound;
wherein, the selectivity of said heterocyclic compound is greater than 95%,
wherein, said nitrogen containing heterocyclic compounds are oxazoline compounds.

2. The process as claimed in claim 1, wherein said metal catalyst is selected from Cu, Al, Zr, Zn, Mg, Ba, Ru, Pd, Pt or combinations thereof.

3. The process as claimed in claim 1, wherein said metal catalyst is selected from combinations of Cu- Al, Cu- Zr or Cu- Mg, Ru-C, Ru/C, Ru/s-ZrO₂,Ru/γ-Al₂O₃.

4. The process as claimed in claim 1, wherein the ratio of glycerol: ammonia is in the range of 1:1 to 1:3.

5. The process as claimed in claim 1, wherein step (a) of said process optionally comprises addition of water to a mixture of glycerol and solid acid metal catalyst.

6. The process as claimed in claim 1, wherein said stirring in step (b) is carried out at temperature in the range of 10°C to 20°C.

7. The process as claimed in claim 1, wherein said process is carried out via reactive distillation or batch mode.

## Patentansprüche

1. Ein Ein-Topf-Verfahren zur Synthese von stickstoffhaltigen heterocyclischen Verbindungen aus Glycerin oder Bio-Glycerin unter Verwendung von festen sauren Metallkatalysatoren, umfassend die Schritte von:
a. Rühren der Mischung aus Glycerin und festem saurem Metallkatalysator bei einer Temperatur im Bereich von 180 bis 240 °C über einen Zeitraum im Bereich von 4 bis 6 Stunden, um Acetol zu erhalten,
b. Abkühlen des Reaktionsgemischs aus Schritt (a) auf eine Temperatur im Bereich von 0 °C bis 50 °C und Zugabe von Ammoniaklösung, gefolgt von Rühren des Gemischs über den Zeitraum im Bereich von 1 bis 10 Stunden, um eine stickstoffhaltige heterocyclische Verbindung zu erhalten,
wobei die Selektivität der heterocyclischen Verbindung größer als 95 % ist, wobei die stickstoffhaltigen heterocyclischen Verbindungen Oxazolinverbindungen sind.

2. Verfahren nach Anspruch 1, wobei der Metallkatalysator ausgewählt ist aus Cu, Al, Zr, Zn, Mg, Ba, Ru, Pd, Pt oder Kombinationen davon.

3. Verfahren nach Anspruch 1, wobei der Metallkatalysator ausgewählt ist aus Kombinationen von Cu-Al, Cu-Zr oder Cu-Mg, Ru-C, Ru/C, Ru/s-ZrO₂, Ru/γ-Al₂O₃.

4. Verfahren nach Anspruch 1, wobei das Verhältnis von Glycerin zu Ammoniak im Bereich von 1:1 bis 1:3 liegt.

5. Verfahren nach Anspruch 1, wobei Schritt (a) des Verfahrens gegebenenfalls die Zugabe von Wasser zu einem Gemisch aus Glycerin und festem saurem Metallkatalysator umfasst.

6. Verfahren nach Anspruch 1, wobei das Rühren in Schritt (b) bei einer Temperatur im Bereich von 10 °C bis 20 °C durchgeführt wird.

7. Verfahren nach Anspruch 1, wobei das Verfahren durch Reaktivdestillation oder Chargenbetrieb ausgeführt wird.

## Revendications

1. Procédé monotope destiné à la synthèse de composés hétérocycliques contenant de l'azote à partir de glycérol ou de bioglycérol utilisant des catalyseurs métalliques acides solides comprenant les étapes :
a. d'agitation du mélange de glycérol et de catalyseur métallique acide solide à une température dans la plage de 180 à 240 °C pendant une période dans la plage de 4 à 6 heures pour obtenir de l'acétol ;
b. de refroidissement du mélange réactionnel de l'étape (a) à une température dans la plage de 0 °C à 50 °C et d'ajout d'une solution d'ammoniaque suivi par l'agitation du mélange pendant la période dans la plage de 1 à 10 heures pour obtenir un composé hétérocyclique contenant de l'azote ;
dans lequel, la sélectivité dudit composé hétérocyclique est supérieure à 95 %, dans lequel, lesdits composés hétérocycliques contenant de l'azote sont des composés oxazoline.

2. Procédé selon la revendication 1, dans lequel ledit catalyseur métallique est sélectionné parmi les Cu, Al, Zr, Zn, Mg, Ba, Ru, Pd, Pt ou des combinaisons de ceux-ci.

3. Procédé selon la revendication 1, dans lequel ledit catalyseur métallique est sélectionné parmi des combinaisons de Cu- Al, Cu- Zr ou Cu- Mg, Ru-C, Ru/C, Ru/s-ZrO₂,Ru/γ-Al₂O₃.

4. Procédé selon la revendication 1, dans lequel le rapport glycérol/ammoniaque est dans la plage de 1/1 à 1/3.

5. Procédé selon la revendication 1, dans lequel l'étape (a) dudit procédé comprend facultativement l'ajout d'eau à un mélange de glycérol et de catalyseur métallique acide solide.

6. Procédé selon la revendication 1, dans lequel ladite agitation dans l'étape (b) est réalisée à une température dans la plage de 10 °C à 20 °C.

7. Procédé selon la revendication 1, dans lequel ledit procédé est réalisé par le biais d'une distillation réactive ou d'un mode par lots.
